# EUROPEAN PATENT SPECIFICATION

(11) **EP 3 208 737 B1**
(45) Date of publication and mention of the grant of the patent: **22.06.2022**
(21) Application number: 16305192.3
(22) Date of filing: 19.02.2016
(51) Int. Cl.: G06F 21/62, A61B 3/11, A61B 5/107

(54) **METHOD FOR PROVIDING A SET OF DATA RELATIVE TO A WEARER OF AN OPHTHALMIC EQUIPMENT AND METHOD FOR DETERMINING THE OPHTHALMIC EQUIPMENT BASED ON THE SET OF DATA**
VERFAHREN ZUR BEREITSTELLUNG EINES DATENSATZES IM ZUSAMMENHANG MIT EINEM TRÄGER EINER OPHTHALMISCHEN VORRICHTUNG UND VERFAHREN ZUR BESTIMMUNG DER OPHTHALMISCHEN VORRICHTUNG BASIEREND AUF DEM DATENSATZ
PROCÉDÉ POUR FOURNIR UN ENSEMBLE DE DONNÉES RELATIVES À L'UTILISATEUR D'UN ÉQUIPEMENT OPHTALMIQUE ET PROCÉDÉ POUR DÉTERMINER L'ÉQUIPEMENT OPHTALMIQUE SUR LA BASE DE L'ENSEMBLE DE DONNÉES

(43) Date of publication of application: 23.08.2017
(73) Proprietor: Essilor International, 94220 Charenton-le-Pont (FR)
(72) Inventor: CLARA, Philippe, 94227 CHARENTON-LE-PONT CEDEX (FR)
(74) Representative: Cabinet Novitech

(56) References cited:
- WO-A1-2015/011286
- JP-A- 2002 078 679
- US-A1- 2008 146 892
- US-A1- 2010 107 219
- US-A1- 2013 231 941
- US-A1- 2014 129 259
- US-A1- 2014 275 851
- US-B1- 6 445 300

## Description

### FIELD OF THE INVENTION

The invention relates to a method for providing a set of data relative to a wearer of an ophthalmic equipment and/or to at least part of the ophthalmic equipment adapted to the wearer to a data server.

The invention further relates to a method for determining the ophthalmic equipment adapted to the wearer based at least on the set of data associated with an identifier and stored on the data server.

### BACKGROUND OF THE INVENTION

Usually, an Eye-Care Professional (ECP) may realize measurements on a wearer so as to provide a set of data relative to the wearer and/or to an ophthalmic equipment adapted to the wearer and to determine the ophthalmic equipment. However, such measurements are provided with expensive and bulky systems. Moreover, such measurements are time consuming for the ECP.

Furthermore, the measurements on the wearer may be realized with measurements applications installed on a computer, on a tablet, or on a smartphone. However, in such applications, the measured data are not protected, namely are accessible on the data server on which the data are stored. Therefore, the privacy of the wearer lacks protection.

Hence, there is a need for a method for providing data relative to a wearer with respect to the privacy of the wearer and with cheaper facilities.

One object of the invention is to provide such a method. General methods of data protection during data exchange are disclosed by the following documents: US6445300, JP2002078679, US2013/231941, US2014/129259, WO2015/011286, US2014/275851, US2008/146892 and US2010/107219.

### SUMMARY OF THE INVENTION

To this end, the invention proposes a method, implemented by computer means, for providing a set of data relative to a wearer of an ophthalmic equipment and/or to at least part of the ophthalmic equipment to a data server, the set of data being intended to be used to determine the ophthalmic equipment adapted to the wearer, the method comprising:
- a set of data receiving step, during which the set of data relative to the wearer and/or to at least part of the ophthalmic equipment is received from a first user, the set of data comprising a private data segment and a public data segment, the private data segment being protected by a private key providing an access to the data of the private data segment;
- a set of data storing step, during which the set of data is stored on the data server and associated with an identifier; and
- an identifier sending step, during which the identifier associated with the stored set of data is sent to a second user,wherein the data of the private data segment are encrypted with a private encryption key, and wherein the private encryption key and the private key are different.

Advantageously, the method of the invention allows protecting the privacy of the wearer and the safety of the wearer data, in particular thanks to the use of an identifier and of a private data segment protected by a private key.

Moreover, receiving a set of data from a first user and sending an identifier associated with said set of data to a second user allows reducing the cost and the time necessary for providing data relative to a wearer and/or to a part of the ophthalmic equipment adapted to the wearer.

According to embodiments, the method for providing a set of data according to the invention may further comprise one or several of the following features according to any possible combination:
- the method further comprises a set of data sending step, during which the public data segment and the private data segment are sent to the second user upon reception of a request comprising at least the identifier; and/or
- the method further comprises a public data segment and a private data segment sending step, during which the public data segment is sent to the second user upon reception of a request comprising at least the identifier, and during which the private data segment is sent to the second user upon reception of a request comprising at least the identifier and the private key; and/or
- the method further comprises an updating step, during which at least a data segment among the private data segment and the public data segment is updated; and/or
- during the set of data receiving step, the method comprises a set of data sorting step, during which the data of the set of data are sorted into a private data segment and a public data segment; and/or
- the private data segment comprises at least one data; and/or
- prior to the set of data receiving step, the method comprises a parameter measuring step, during which at least a parameter relative to the wearer and/or a parameter relative to at least part of the ophthalmic equipment is measured, the set of data comprising the at least one measured parameter; and/or
- at least a part of the data of the set of data is measured based on a 3D modeling of the face of the wearer and/or based on a two-dimensional image of the face of the wearer; and/or
- the private data segment comprises at least data relative to the name of the wearer and/or to the date of birth of the wearer and/or to the gender of the wearer and/or to laterality of the wearer and/or to the physiological characteristics of the wearer and/or to a color of an iris of at least one of the eye of the wearer and/or to a position of a center of rotation of at least one of the eye of the wearer in respect with the wearer anatomy and/or to a near vision inter-pupillary distance for the eyes of the wearer and/or to a reading distance for at least one eye of the wearer and/or to a lowering angle of gaze reading direction for at least one eye of the wearer; and/or
- the ophthalmic equipment comprises a frame, and wherein the private data segment comprises at least data relative to a vertex distance for one eye of the wearer when the frame is worn by the wearer and/or to a frame wrap angle of the frame when the frame is worn by the wearer and/or to a frame pantoscopic angle of the frame when the frame is worn by the wearer; and/or
- the public data segment comprises at least data relative to a far vision inter-pupillary distance for the eyes of the wearer; and/or
- the ophthalmic equipment comprises a frame, and wherein the public data segment comprises at least a frame boxing height and width and/or an optical height of at least one eye of the wearer and/or to a position of a center of rotation of at least one of the eye of the wearer in respect with the frame when the frame is worn by the wearer; and/or
- the data of the private data segment are encrypted with a private encryption key; and/or
- the private encryption key and the private key are different; and/or
- the private key and the private encryption key are identical.

Advantageously, the updating step of the method of the invention allows expanding and adjusting the set of data, and thus allows taking into account the correction of a prescription.

The invention further relates to a computer program product comprising one or more stored sequences of instructions that are accessible to a processor and which, when executed by the processor, causes the processor to carry out the steps of the method for providing a set of data according to the invention.

The invention further relates to a system for providing a set of data relative to a wearer of an ophthalmic equipment and/or to at least part of the ophthalmic equipment to a data server comprising a processor adapted to store one or more sequences of instruction and to carry out at least one of the steps of the method for providing a set of data according to the invention.

The invention further relates to a computer readable storage medium having a program recorded thereon, where the program makes the computer execute the steps of the method for providing a set of data according to the invention.

The invention further relates to a method, implemented by computer means, for determining an ophthalmic equipment adapted to a wearer based at least on a set of data associated with an identifier and stored on a data server, the set of data being relative to the wearer and/or to at least part of the ophthalmic equipment and comprising a public data segment and a private data segment, the private data segment being protected by a private key providing an access to the data of the private data segment, the method comprising:
- a data receiving step, during which at least a portion of the set of data relative to the wearer and/or to at least part of the ophthalmic equipment is received upon reception of at least the identifier, the portion of the set of data comprising at least the public data segment;
- an ophthalmic equipment determining step, during which an ophthalmic equipment is determined based on the received portion of the set of data.

Advantageously, the method of the invention allows determining an ophthalmic equipment personalized to the wearer.

Additionally, the method of the invention allows reducing the cost and the time of production of the ophthalmic equipment adapted to the wearer, especially thanks to the reduction of the cost and the time of acquisition of the data relative to the wearer and/or to a part of the ophthalmic equipment.

According to embodiments, the method for determining an ophthalmic equipment according to the invention may further comprise one or several of the following features according to any possible combination:
- the private data segment comprises at least a first private data segment and a second private data segment, the first private data segment comprising the second data segment, the first private data segment being protected by a first private key providing an access to the data of the first private data segment, the second private data segment being protected by a second private key providing an access to the data of the second private data segment, the first private key being different from the second private key, and wherein during the ophthalmic equipment determining step, the ophthalmic equipment is determined based:
   - on the public data segment and on the first private data segment upon reception of the first private key; or
   - on the public data segment and on the second data segment upon reception of the second private key; and/or
- during the data receiving step, the portion of the set of data further comprises the private data segment, and during the ophthalmic equipment determining step, the ophthalmic equipment is determined based on the public data segment and at least part of the private data segment upon reception of the private key associated to the part of the private data segment; and/or
- during the data receiving step, the portion of the set of data further comprises at least a part of the private data segment upon reception of a private key associated to the part of the private data segment, and during the ophthalmic equipment determining step, the ophthalmic equipment is determined based on the received portion of the set of data comprising public data segment and the at least part of the private data segment.

The invention further relates to a computer program product comprising one or more stored sequences of instructions that are accessible to a processor and which, when executed by the processor, causes the processor to carry out the steps of the method for determining an ophthalmic equipment according to the invention.

The invention further relates to a system for determining an ophthalmic equipment adapted to a wearer based at least on a set of data associated with an identifier and stored on a data server comprising a processor adapted to store one or more sequences of instruction and to carry out at least one of the steps of the method for determining an ophthalmic equipment according to the invention.

The invention further relates to a computer readable storage medium having a program recorded thereon, where the program makes the computer execute the steps of the method for determining an ophthalmic equipment according to the invention.

Unless specifically stated otherwise, as apparent from the following discussions, it is appreciated that throughout the specification discussions utilizing terms such as "computing", "calculating", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. This apparatus may be specially constructed for the desired purposes, or it may comprise a general purpose computer or Digital Signal Processor ("DSP") selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storing electronic instructions, and capable of being coupled to a computer system bus.

The processes and displays presented herein are not inherently related to any particular computer or other apparatus. Various general purpose systems may be used with programs in accordance with the teachings herein, or it may prove convenient to construct a more specialized apparatus to perform the desired method. The desired structure for a variety of these systems will appear from the description below.

In addition, embodiments of the present invention are not described with reference to any particular programming language. It will be appreciated that a variety of programming languages may be used to implement the teachings of the inventions as described herein.

Another object of the invention is a network data-processing device comprising means for:
- receiving from a first user, a set of data relative to a wearer of an ophthalmic equipment and/or to at least part of the ophthalmic equipment, the set of data comprising a private data segment and a public data segment, the private data segment being protected by a private key providing an access to the data of the private data segment;
- storing the set of data and associating the set of data with an identifier;
- sending the identifier to a second user.

According to embodiments, the network data-processing device according to the invention may further comprise one or any possible combination of the following features:
- the network data-processing device further comprising means for:
   - receiving from the second user, a request comprising at least the identifier;
   - sending the public data segment and the private data segment to the second user upon reception of the request; and/or
- the network data-processing device further comprising means for:
   - receiving from the second user, a request comprising at least the identifier and a private key;
   - sending the public data segment of the set of data associated with the identifier and the private data segment of the set of data associated with the identifier and with the private key to the second user upon reception of the request.

The invention is defined in the appended claims.

### BRIEF DESCRIPTION OF THE DRAWINGS

Other characteristics and advantages of the invention will become more apparent from the claims and from the following description of some embodiments given by way of example without limitation with reference to the drawings, in which:
- Figure 1 is a flowchart of the different steps of a method for providing a set of data according to the invention,
- Figure 2 is a networked data processing device performing the method for providing a set of data according to the invention,
- Figure 3 represents a network data-processing device according to the invention, and
- Figure 4 is a flowchart of the different steps of a method for determining an ophthalmic equipment according to the invention.

Elements in the figures are illustrated for simplicity and clarity and have not necessarily been drawn to scale. For example, the dimensions of some of the elements in the figure may be exaggerated relative to other elements to help improve the understanding of the embodiments of the present invention.

### DETAILED DESCRIPTION OF PREFERRED EMBODIMENTS

The invention relates to a method, for example implemented by computer means, for providing a set of data relative to a wearer of an ophthalmic equipment and/or to a part of the ophthalmic equipment to a data server.

The set of data is intended to be used to determine the ophthalmic equipment adapted to the wearer.

The ophthalmic equipment may be, for instance, an ophthalmic lens, a pair of ophthalmic lenses or spectacle ophthalmic lenses. In particular, the ophthalmic equipment is intended to be worn by the wearer.

The term *"ophthalmic lens"* is to be understood to mean any type of known lens intended to be supported by a wearer's face. The term can refer to non-corrective lenses, corrective lenses, such as progressive addition lenses, unifocal, occupational or multifocal lenses. The term can also refer to said ophthalmic lenses which could present at least one added value such as, for example, tint, photochromism, polarization filtering, electrochromism, antireflective properties, antiscratch properties. The lens may be also a lens for information glasses, wherein the lens comprises means for displaying information in front of the eye.

The data server may be, for instance a cloud, or a database of a distant entity such as a server connected to the public Internet.

The set of data is intended to be used to determine the ophthalmic equipment adapted to the wearer.

The ophthalmic equipment may be, for instance, an ophthalmic lens, a pair of ophthalmic lenses or spectacle ophthalmic lenses. In particular, the ophthalmic equipment is intended to be worn by the wearer.

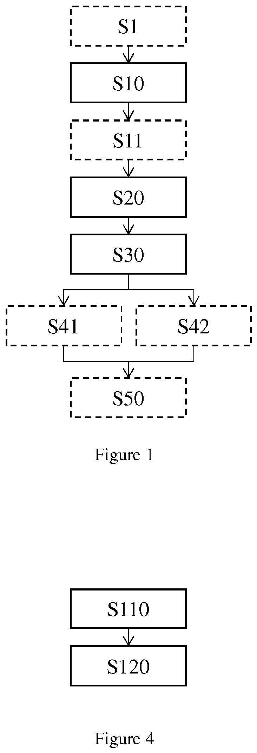

The steps of the method of the invention are illustrated on figure 1. The method for providing a set of data comprises:
- a set of data receiving step S10,
- a set of data storing step S20, and
- an identifier sending step S30.

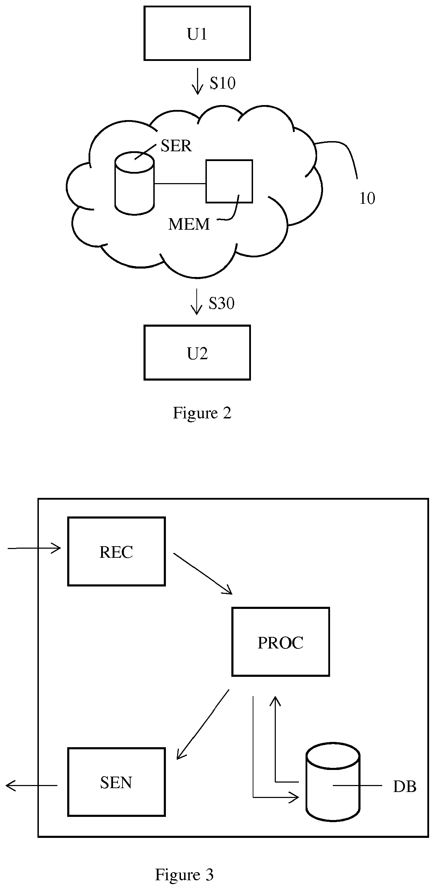

As illustrated on figure 2, the method according to the invention may be performed by a networked data processing device 10 comprising a server SER and a storage unit MEM.

The networked data processing device 10 is connected at least to a first user U1 and to a second user U2. The networked data processing device 10 may be connected to both of the first and second user over the internet.

During the set of data receiving step S10, the set of data is received, for example by the networked data processing device 10, from the first user. The first user may be the wearer or an ECP measuring data on the wearer or providing the data of the set of data.

The set of data is divided into a private data segment and a public data segment. In other words, a first part of the data of the set is public data and a second part of the data of the set is private data, the second part of the data being complementary to the first part of the data.

In particular, during the set of data receiving step S10, the method may comprise a set of data sorting step S11, during which the data of the set of data are sorted into the private data segment and the public data segment.

Some data may be redundant. More precisely, the private data segment and the public data segment may both comprise the same data. In this case, the data comprised in the private data segment is advantageously more accurate than the data comprised in the public data segment.

The private data segment is protected by a private key providing an access to the data of the private data segment. In particular, the private key is generated either by the data server or by the first user. The private data segment preferably comprises at least one data.

According to an embodiment of the invention, the private data segment comprises data relative to the privacy of the wearer, for instance the name of the wearer and/or the date of birth of the wearer and/or the gender of the wearer.

The private data segment may further comprise data relative to the laterality of the wearer and/or to the physiological characteristics of the wearer and/or to the color of the iris of the eyes of the wearer and/or to a near vision inter-pupillary distance for the eyes of the wearer.

The private data segment may further comprise data relative to the position of the center of rotation of at least one eye of the wearer in respect with the wearer anatomy. The center of rotation of an eye may correspond to a standard position, or may be determined by direct measurements. In particular, the document US 7,794,085 B2 describes a method for determining the center of rotation of the eye.

The private data segment may further comprise data relative to a reading parameter, for instance a reading distance for at least one eye of the wearer and/or a lowering angle of gaze reading direction for at least one eye of the wearer.

Moreover, when the ophthalmic equipment comprises a frame, the private data segment may comprise data relative to a vertex distance for one eye of the wearer when the frame is worn by the wearer and/or to a frame wrap angle of the frame when the frame is worn by the wearer and/or to a frame pantoscopic angle of the frame when the frame is worn by the wearer.

In addition, when the ophthalmic equipment comprises a frame, the private data segment may also comprise data relative to the bridge of the frame and/or to geometrical data representative of the geometry of the area of the wearer's head supporting the ophthalmic equipment.

According to an embodiment of the invention, the public data segment comprises data relative to a far vision inter-pupillary distance for the eyes of the wearer.

The public data segment may also comprise wearer's prescription data including for example the spherical power, the astigmatism, the cylinder axis of the astigmatism, or the power addition.

In addition, when the ophthalmic equipment comprises a frame, the public data segment may comprise a frame boxing height and width and/or a position of a center of rotation of at least one of the eye of the wearer in respect with the frame when the frame is worn by the wearer. Said position of a center of rotation may be expressed in a referential linked with one wearer's eye or linked with the ophthalmic equipment when worn by the wearer.

The public data segment may further comprise an optical height of at least one eye of the wearer. The optical height is defined as the distance between the pupil of an eye of the wearer in the primary gazing direction and the inferior edge of the frame when the frame is worn by the wearer.

According to an embodiment of the invention, the private data segment may comprise a first private data segment protected by a first private key providing an access to the data of the first private data segment and a second private data segment protected by a second private key providing an access to the data of the second private data segment. In particular, the first private key is different from the second private key.

The first private data segment may comprise the second data segment, or the second data segment may be complementary to the first data segment.

The public data segment and the first private data segment comprise the data for determining a first ophthalmic equipment, and the public data segment and the second private data segment comprise the data for determining a second ophthalmic equipment, the first ophthalmic equipment being different from the second ophthalmic equipment.

For instance, if the first private data segment comprises more data than the second private data segment, the first ophthalmic equipment is intended to be more suitable for the wearer than the second ophthalmic equipment.

The public data segment comprises the minimal data for determining an ophthalmic equipment. In particular, the first or the second ophthalmic equipment is intended to be more personalized for the wearer than the ophthalmic equipment determined only based on the public data.

More generally, the private data segment can comprise a plurality of private data sub-segment, each private data sub-segment being protected by a private key providing an access to the data of said private data sub-segment.

During the set of data storing step S20, the set of data is stored on the data server, for instance stored by the networked data processing device 10, for example in the storing unit MEM. When stored, the set of data is associated with an identifier identifying the wearer of the ophthalmic equipment or part of the ophthalmic equipment. More precisely, the identifier allows having access to the set of data on the data server. Advantageously, the identifier is associated with a PIN code to ensure the wearer's privacy.

Advantageously, the set of data associated with an identifier and comprising a private data segment allows protecting the privacy of the wearer, and thus increasing the safety of the wearer data.

During the identifier sending step S30, the identifier associated with the stored set of data is sent to a second user. The second user may be the same user or a different user as the first user. The second user may be, for example, the wearer, or the ECP, or an ophthalmic lens manufacturer, or an ophthalmic lens designer. The identifier may be provided to the second user electronically, for instance like a Quick-Response (QR) code, or a barcode, or a web link containing the identifier sent by text messages.

Advantageously, the first user is the wearer and the second user is the ECP so as to reduce the cost and the time necessary for providing the set of data. For instance, the wearer may provide the set of data with a measurement application installed on his/her computer, or on his/her tablet, or on his/her smartphone. In this case, the wearer does not have to go to the ECP for providing the measurements, and the ECP may receive the set of data of the wearer without spending time taking the measurements on the wearer. Moreover, the measurement application may replace usual devices allowing to provide the measurements, and thus allows reducing the cost necessary for providing the set of data.

According to an embodiment of the invention, the method comprises a set of data sending step S41, during which the public data segment and the private data segment are sent to the second user upon reception of a request comprising at least the identifier.

In other words, during the implementation of the method of the invention, the user sends a request comprising the identifier, and upon reception of the request, the user receives the set of data, namely the public data segment and the private data segment, associated with the identifier. More precisely, the request allows the user receiving the set of data.

Upon reception of the set of data, the user may access, and therefore use, the data of the public data segment.

At this stage, the private data segment is still protected by a private key but is accessible and thus usable upon reception of a request comprising at least the private key.

According to a variant, the method comprises a public data segment and a private data segment sending step S42, during which the public data segment is sent to the second user upon reception of a request comprising at least the identifier, and during which the private data segment is sent to the second user upon reception of a request comprising at least the identifier and the private key.

In other words, in operation of the method of the invention, the user sends a first request comprising the identifier, and upon reception of the first request, the user receives the public data segment associated with the identifier.

Then, the user sends a second request comprising at least the private key, and upon reception of the second request, the user receives the private data segment associated with the identifier and the private key.

More precisely, the first request allows the user receiving only the public data segment, while the second request allows the user receiving only the private data segment.

Unlike the preceding embodiment, the private data segment is received only upon reception of a request comprising at least the private key.

According to an embodiment of the invention compatible with the previous ones, the data of the private data segment are encrypted with a private encryption key. More precisely, the private key allows receiving the private data segment, the private data segment being previously encrypted, and the private encryption key allows decrypting the private data segment. Advantageously, a previous encryption of the private data segment allows increasing the safety of the data of the private data segment.

The private key may be identical to the private encryption key.

Preferably, the private encryption key and the private key are different. More precisely, the data of the private data segment are twice protected. Advantageously, a private encryption key different from the private key allows increasing the safety of the data, and thus the privacy of the wearer.

According to an embodiment of the invention, the method further comprises an updating step S50, during which at least a data segment among the private data segment and the public data segment is updated.

In other words, the set of data, the private data segment or the public data segment may be updated. More precisely, data relative to the wearer, for instance the wearer's prescription, may vary with time, and thus such data need to be updated.

Additionally, new data may be added to the private data segment and/or the public data segment.

In addition, data of the private data segment may be removed from the private data segment and added to the public data segment. In other words, data of the private data segment may be transferred to the public data segment. In the same way, data of the public data segment may be transferred to the private data segment.

Advantageously, the updating step allows completing and correcting the set of data so as to obtain, for instance, an ophthalmic equipment more suitable for a wearer.

According to an embodiment of the invention, prior to the set of data receiving step S10, the method comprises a parameter measuring step S1, during which at least a parameter relative to the wearer and/or a parameter relative to a part of the ophthalmic equipment is measured. The set of data comprises the measured parameters.

The measured parameters may be, for example, physiological characteristics of the wearer, the laterality of the wearer, the inter-pupillary distance of the wearer, the color of the iris of the eyes of the wearer, the position of the center of rotation of an eye of the wearer, a reading distance for an eye of the wearer, a lowering angle of gaze reading direction for an eye of the wearer, the frame pantoscopic angle when the frame is worn by the wearer, the frame wrap angle when the frame is worn by the wearer, the bridge of the frame, the vertex distance of an eye of the wearer when the frame is worn by the wearer, the frame boxing height and width, the optical height of an eye of the wearer, geometrical data representative of the geometry of the area of the wearer's head supporting the ophthalmic equipment. Said position of a center of rotation may be expressed in a referential linked with one wearer's eye or linked with the ophthalmic equipment when worn by the wearer.

A part of the data of the set of data may be measured based on a display of a three-dimensional (3D) modeling of the face of the wearer. In particular, the head area of the wearer and the areas close or in contact with the frame when worn by the wearer, for example the nose or ears temple, are modeled. The measured data may depend on the mesh of the model.

A part of the data of the set of data may also be measured based on a two-dimensional (2D) image of the face of the wearer. More precisely, data may be measured based on a plurality of 2D images of the face of the wearer, the images corresponding to different angle of view of the face of the wearer.

In particular, the face of the wearer may be displayed in 3D or in 2D with and without an ophthalmic equipment. The ophthalmic equipment worn by the wearer may be the actual ophthalmic equipment of the wearer, or may be a virtual ophthalmic equipment superposed with the 3D modeling or with the 2D image of the face of a wearer, or may be a frame worn by the wearer. More precisely, the measured parameter may be deduced from the difference of a parameter between the display in 3D or in 2D of the face of the wearer with and without the ophthalmic equipment. A 3D modeling or a 2D image of the face of a wearer may be acquired by a first device, such as a camera of a smartphone or of a tablet, or a webcam of a computer.

Such modeling or image may be then send to a second device for the measuring step. In other words, an image acquisition device acquires images or videos of the wearer. The image acquisition device is in relation with an image computing device. The exchange of information between the image acquisition device and the image computing device is done remotely in order to provide a high power computing.

The image computing device computes the information received from the image acquisition device for measuring the parameters.

The image computing device may send a request to the image acquisition device for acquiring new images or videos, with new viewing angle or with zooms on specific parts of the face of the wearer.

The measurements may be provided by the wearer, for instance via an application on a smartphone, on a tablet or on a computer, or by ECP, for instance via usual measuring device, or via an application on a smartphone, on a tablet or on a computer.

As illustrated on figure 3, the invention also relates to a network data-processing device configured to implement the method for providing a set of data according to the invention. The network data-processing device comprises at least a receiving module REC, a processing module PROC, a storing module DB, for example a data-base type, and a sending module SEN.

The receiving module REC is configured so as to receive at least requests and data from distant entities.

The processing module PROC is configured so as to process the requests and data received by the receiving module REC.

The sending module is configured so as to send data to distant entities.

First, the receiving module REC receives, from a first user, a set of data relative to a wearer of an ophthalmic equipment and/or to at least part of the ophthalmic equipment. The set of data comprises a public data segment and a private data segment protected by a private key providing an access to the data of the private data segment.

The set of data received by the receiving module REC is transferred to the processing module PROC. The processing module associates the set of data with an identifier identifying the wearer of the ophthalmic equipment and/or part of the ophthalmic equipment to be provided to the wearer. The set of data and the identifier are stored in the storing module DB.

The processing module PROC has the sending module SEN send the identifier to a second user.

According to an embodiment of the invention, the receiving module REC is arranged to receive a request comprising at least the identifier, for example from the second user, and to transfer such request to the processing module PROC. The processing module PROC is configured so as to, upon reception of the request, extract and have the sending module SEN send the public data segment and the private data segment, for example to the second user.

According to a variant, the receiving module REC is arranged to receive a request comprising at least the identifier, for example from the second user, and to transfer such request to the processing module PROC. The processing module PROC is configured so as to, upon reception of the request, extract and have the sending module SEN send the public data segment associated with the identifier, for example to the second user. Moreover, the receiving module REC is arranged to receive a request comprising at least the identifier and a private key, from the second user, and to transfer such request to the processing module PROC. The processing module PROC is configured so as to, upon reception of the request, extract and have the sending module SEN send the public data segment associated with the identifier and the private data segment of the set of data associated with the identifier and with the private key, to the second user.

The invention further relates to a method, for example implemented by computer means, for determining an ophthalmic equipment adapted to a wearer based at least on the set of data associated with the identifier and stored on the data server as defined previously.

The steps of the method of the invention are illustrated on figure 4. The method for determining an ophthalmic equipment comprises:
- a data receiving step S110, and
- an ophthalmic equipment determining step S120.

During the data receiving step S 110, at least a portion of the set of data is received upon reception of at least the identifier. The portion of the set of data comprises at least the public data segment. Advantageously, the portion of the set of data further comprises the private data segment.

During the ophthalmic equipment determining step S120, an ophthalmic equipment is determined based on the received portion of the set of data.

Advantageously, an ophthalmic equipment determined based on both the public and private data segments is more adapted to the wearer than an ophthalmic equipment determined based only on the public data segment.

According to an embodiment of the invention, the private data segment comprises a first private data segment and a second private data segment as defined previously.

During the ophthalmic equipment determining step S120, the ophthalmic equipment is determined based:
- on the public data segment and on the first private data segment upon reception of the first private key; or
- on the public data segment and on the second data segment upon reception of the second private key.

In other words, the ophthalmic equipment is determined based on the public data segment and on a private data segment upon reception of a private key associated with a private data segment.

According to an embodiment of the invention, during the data receiving step S110, the portion of the set of data further comprises the private data segment. During the ophthalmic equipment determining step S120, the ophthalmic equipment is determined based on the public data segment and on at least a part of the private data segment upon reception of the private key associated to the part of the private data segment.

In other words, in operation of the method of the invention, a user sends a request comprising the identifier, and upon reception of the request, the user receives the set of data, namely the public data segment and the private data segment, associated with the identifier. More precisely, the request allows the user receiving the set of data.

Upon reception of the set of data, the private data segment is still protected, and thus the ophthalmic equipment may be determined only based on the data of the public data segment.

Then, upon reception of a request comprising at least a private key associated to at least a part of the private data segment, the ophthalmic equipment is determined based on the public data segment and on the part of the private data segment.

According to a variant, during the data receiving step S110, the portion of the set of data further comprises at least a part of the private data segment upon reception of a private key associated to the part of the private data segment. During the ophthalmic equipment determining step S120, the ophthalmic equipment is determined based on the received portion of the set of data comprising public data segment and the at least part of the private data segment.

In other words, during the implementation of the method of the invention, a user sends a first request comprising the identifier, and upon reception of the first request, the user receives the public data segment associated with the identifier.

Then, the user sends a second request comprising at least a private key, and upon reception of the second request, the user receives the private data segment associated with the private key.

Upon reception of only the public data segment, the ophthalmic equipment may be determined based only on the public data segment, and upon reception of the public data segment and of a part of the private data segment, the ophthalmic equipment is determined based on both the public data segment and on the part of the private data segment.

Unlike the preceding embodiment, the private data segment is received only upon reception of a request comprising at least the private key.

The invention has been described above with the aid of embodiments without limitation of the general inventive concept. Moreover, the embodiments of the invention may be combined without any restriction.

Many further modifications and variations will suggest themselves to those skilled in the art upon making reference to the foregoing illustrative embodiments, which are given by way of example only and which are not intended to limit the scope of the invention, that being determined solely by the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

## Claims

1. A method, implemented by computer means, for providing a set-of-data relative to a wearer of an ophthalmic equipment and/or to at least part of the ophthalmic equipment to a data server, the set-of-data being intended to be used to determine the ophthalmic equipment adapted to the wearer, the method comprising:
- a **set-of-data receiving step** (S10), during which the set-of-data relative to the wearer and/or to at least part of the ophthalmic equipment is received from a first user, the set-of-data comprising a private data segment and a public data segment, the private data segment being protected by a private key providing an access to the data of the private data segment;
- a **set-of-data storing step** (S20), during which the set-of-data is stored on the data server and associated with an identifier; and
- an **identifier sending step** (S30), during which the identifier associated with the stored set-of-data is sent to a second user,
**characterised in that**
the data of the private data segment are encrypted with a private encryption key, and
wherein the private encryption key and the private key are different.

2. Method, according to claim 1, wherein the method further comprises a **set-of-data sending step** (S41), during which the public data segment and the private data segment are sent to the second user upon reception of a request comprising at least the identifier.

3. Method, according to claim 1, wherein the method further comprises a **public data segment and a private data segment sending step** (S42), during which the public data segment is sent to the second user upon reception of a request comprising at least the identifier, and during which the private data segment is sent to the second user upon reception of a request comprising at least the identifier and the private key.

4. Method according to any of the preceding claims, wherein the method further comprises an **updating step** (S50), during which at least a data segment among the private data segment and the public data segment is updated.

5. Method according to any of the preceding claims, wherein prior to the set-of-data receiving step (S10), the method comprises a **parameter measuring step** (S1), during which at least a parameter relative to the wearer and/or a parameter relative to at least part of the ophthalmic equipment is measured, the set-of-data comprising the at least one measured parameter.

6. Method according to claim 5, wherein at least a part of the data of the set-of-data is measured based on a 3D modeling of the face of the wearer and/or based on a two-dimensional image of the face of the wearer.

7. Method according to any of the preceding claims, wherein the private data segment comprises at least data relative to the name of the wearer and/or to the date of birth of the wearer and/or to the gender of the wearer and/or to laterality of the wearer and/or to the physiological characteristics of the wearer and/or to a color of an iris of at least one of the eye of the wearer and/or to a position of a center of rotation of at least one of the eye of the wearer in respect with the wearer anatomy and/or to a near vision inter-pupillary distance for the eyes of the wearer and/or to a reading distance for at least one eye of the wearer and/or to a lowering angle of gaze reading direction for at least one eye of the wearer.

8. Method according to any of the preceding claims, wherein the ophthalmic equipment comprises a frame, and wherein the private data segment comprises at least data relative to a vertex distance for one eye of the wearer when the frame is worn by the wearer and/or to a frame wrap angle of the frame when the frame is worn by the wearer and/or to a frame pantoscopic angle of the frame when the frame is worn by the wearer.

9. Method according to any of the preceding claims, wherein the public data segment comprises at least data relative to a far vision inter-pupillary distance for the eyes of the wearer.

10. Method according to any of the preceding claims, wherein the ophthalmic equipment comprises a frame, and wherein the public data segment comprises at least a frame boxing height and width and/or an optical height of at least one eye of the wearer and/or to a position of a center of rotation of at least one of the eye of the wearer in respect with the frame when the frame is worn by the wearer.

11. A method, implemented by computer means, for determining an ophthalmic equipment adapted to a wearer based at least on the set-of-data according to claim 1 associated with the identifier and stored on the data server according to claim 1, the method comprising:
- a **data receiving step** (S110), during which at least a portion of the set-of-data relative to the wearer and/or to at least part of the ophthalmic equipment is received upon reception of a request comprising at least the identifier, the portion of the set-of-data comprising at least the public data segment;
- an **ophthalmic equipment determining step** (S120), during which an ophthalmic equipment is determined based on the received portion of the set-of-data.

12. Method according to claim 11, wherein the private data segment comprises at least a first private data segment and a second private data segment, the first private data segment comprising the second data segment, the first private data segment being protected by a first private key providing an access to the data of the first private data segment, the second private data segment being protected by a second private key providing an access to the data of the second private data segment, the first private key being different from the second private key, and wherein during the ophthalmic equipment determining step (S120), the ophthalmic equipment is determined based:
- on the public data segment and on the first private data segment upon reception of the first private key; or
- on the public data segment and on the second data segment upon reception of the second private key.

13. Method according to claim 11 or 12, wherein
during the data receiving step (S110), the portion of the set-of-data further comprises the private data segment, and
during the ophthalmic equipment determining step (S 120), the ophthalmic equipment is determined based on the public data segment and at least part of the private data segment upon reception of the private key associated to the part of the private data segment.

14. Method according to claim 11 or 12, wherein
during the data receiving step (S110), the portion of the set-of-data further comprises at least a part of the private data segment upon reception of a private key associated to the part of the private data segment, and
during the ophthalmic equipment determining step (S 120), the ophthalmic equipment is determined based on the received portion of the set-of-data comprising public data segment and the at least part of the private data segment.

## Patentansprüche

1. Verfahren, das durch Computermittel implementiert wird, um einen Datensatz, der sich auf eine Träger einer ophthalmischen Ausrüstung und/oder wenigstens einen Teil der ophthalmischen Ausrüstung bezieht, an einen Datenserver zu liefern, wobei der Datensatz dazu vorgesehen ist, zum Bestimmen der an den Träger angepassten ophthalmischen Ausrüstung verwendet zu werden, wobei das Verfahren umfasst:
- einen Schritt des Empfangens eines Datensatzes (S10), in dem der Datensatz, der sich auf den Träger und/oder wenigstens einen Teil der ophthalmischen Ausrüstung bezieht, von einem ersten Benutzer empfangen wird, wobei der Datensatz ein privates Datensegment und ein öffentliches Datensegment umfasst, wobei das private Datensegment durch einen privaten Schlüssel geschützt ist, der einen Zugang zu den Daten des privaten Datensegments ermöglicht;
- einen Schritt des Speicherns des Datensatzes (S20), in dem der Datensatz auf dem Datenserver gespeichert und mit einem Kennzeichner verknüpft wird; und
- einen Schritt des Sendens eines Kennzeichners (S30), in dem der Kennzeichner, der mit dem gespeicherten Datensatz verknüpft ist, an einen zweiten Benutzer gesendet wird,
**dadurch gekennzeichnet, dass**
die Daten des privaten Datensegments mit einem privaten Verschlüsselungsschlüssel verschlüsselt werden, und
wobei der private Verschlüsselungsschlüssel und der private Schlüssel verschieden sind.

2. Verfahren gemäß Anspruch 1, wobei das Verfahren ferner einen Schritt des Sendens eines Datensatzes (S41) umfasst, in dem das öffentliche Datensegment und das private Datensegment bei Empfang einer Anfrage, die wenigstens den Kennzeichner enthält, an den zweiten Benutzer gesendet werden.

3. Verfahren gemäß Anspruch 1, wobei das Verfahren ferner einen Schritt des Sendens eines öffentlichen Datensegments und eines privaten Datensegments (S42) umfasst, in dem das öffentliche Datensegment bei Empfang einer Anfrage, die wenigstens den Kennzeichner enthält, an den zweiten Benutzer gesendet wird, und in dem das private Datensegment bei Empfang einer Anfrage, die wenigstens den Kennzeichner und den privaten Schlüssel enthält, an den zweiten Benutzer gesendet wird.

4. Verfahren gemäß einem der vorstehenden Ansprüche, wobei das Verfahren ferner einen Schritt des Aktualisierens (S50) umfasst, in dem wenigstens ein Datensegment aus dem privaten Datensegment und dem öffentlichen Datensegment aktualisiert wird.

5. Verfahren gemäß einem der vorstehenden Ansprüche, wobei das Verfahren vor dem Schritt des Empfangens des Datensatzes (S10) einen Schritt des Messens von Parametern (S1) umfasst, in dem wenigstens ein Parameter bezüglich des Trägers und/oder ein Parameter bezüglich wenigstens eines Teils der ophthalmischen Ausrüstung gemessen werden, wobei der Datensatz den wenigstens einen gemessenen Parameter umfasst.

6. Verfahren gemäß Anspruch 5, wobei wenigstens ein Teil der Daten des Datensatzes basierend auf einer 3D-Modellierung des Gesichts des Trägers und/oder basierend auf einem zweidimensionalen Bild des Gesichts des Trägers gemessen wird.

7. Verfahren gemäß einem der vorstehenden Ansprüche, wobei das private Datensegment wenigstens Daten umfasst, die sich auf den Namen des Trägers und/oder auf das Geburtsdatum des Trägers und/oder auf das Geschlecht des Trägers und/oder auf die Lateralität des Trägers und/oder auf die physiologischen Merkmale des Trägers und/oder auf eine Farbe einer Iris wenigstens eines Auges des Trägers und/oder auf eine Position eines Rotationszentrums wenigstens eines Auges des Trägers in Bezug auf die Anatomie des Trägers und/oder auf einen Nahseh-Pupillenabstand für die Augen des Trägers und/oder auf einen Leseabstand für wenigstens ein Auge des Trägers und/oder auf einen Absenkungswinkel der Blickleserichtung für wenigstens ein Auge des Trägers beziehen.

8. Verfahren gemäß einem der vorstehenden Ansprüche, wobei die ophthalmische Ausrüstung einen Rahmen umfasst und wobei das private Datensegment wenigstens Daten umfasst, die sich auf einen Scheitelabstand für ein Auge des Trägers, wenn der Rahmen vom Träger getragen wird, und/oder auf einen Rahmenumschlingungswinkel des Rahmens, wenn der Rahmen vom Träger getragen wird, und/oder auf einen Rahmenpantoskopwinkel des Rahmens, wenn der Rahmen vom Träger getragen wird, beziehen.

9. Verfahren gemäß einem der vorstehenden Ansprüche, wobei das öffentliche Datensegment wenigstens Daten umfasst, die sich auf einen Weitseh-Pupillenabstand für die Augen des Trägers beziehen.

10. Verfahren gemäß einem der vorstehenden Ansprüche, wobei die ophthalmische Ausrüstung einen Rahmen umfasst und wobei das öffentliche Datensegment wenigstens eine Höhe und Breite des Rahmens und/oder eine optische Höhe wenigstens eines Auges des Trägers und/oder eine Position eines Rotationspunktes wenigstens eines Auges des Trägers in Bezug auf den Rahmen, wenn der Rahmen vom Träger getragen wird, umfasst.

11. Verfahren, das durch Computermittel implementiert wird, um eine an einen Träger angepasste ophthalmische Ausrüstung zu bestimmen, basierend wenigstens auf dem Datensatz gemäß Anspruch 1, der mit dem Kennzeichner verknüpft und auf dem Datenserver gemäß Anspruch 1 gespeichert ist, wobei das Verfahren umfasst:
- einen Schritt des Empfangens von Daten (S110), in dem wenigstens ein Teil des Datensatzes, der sich auf einen Träger und/oder wenigstens einen Teil der ophthalmischen Ausrüstung bezieht, bei Empfang einer Anfrage, die wenigstens den Kennzeichner umfasst, empfangen wird, wobei der Teil des Datensatzes wenigstens das öffentliche Datensegment umfasst;
- einen Schritt des Bestimmens der ophthalmischen Ausrüstung (S120), in dem eine ophthalmische Ausrüstung basierend auf dem empfangenen Teil des Datensatzes bestimmt wird.

12. Verfahren gemäß Anspruch 11, wobei das private Datensegment wenigstens ein erstes privates Datensegment und ein zweites privates Datensegment umfasst, wobei das erste private Datensegment das zweite Datensegment umfasst, wobei das erste private Datensegment durch einen ersten privaten Schlüssel geschützt ist, der einen Zugang zu den Daten des ersten privaten Datensegments ermöglicht, wobei das zweite private Datensegment durch einen zweiten privaten Schlüssel geschützt ist, der einen Zugang zu den Daten des zweiten privaten Datensegments ermöglicht, wobei der erste private Schlüssel vom zweiten privaten Schlüssel verschieden ist und wobei im Schritt des Bestimmens der ophthalmischen Ausrüstung (S120) die ophthalmische Ausrüstung basierend auf folgendem bestimmt wird:
- auf dem öffentlichen Datensegment und auf dem ersten privaten Datensegment bei Empfang des ersten privaten Schlüssels; oder
- auf dem öffentlichen Datensegment und auf dem zweiten privaten Datensegment bei Empfang des zweiten privaten Schlüssels.

13. Verfahren gemäß Anspruch 11 oder 12, wobei im Schritt des Empfangens von Daten (S110) der Teil des Datensatzes ferner das private Datensegment umfasst und im Schritt des Bestimmens der ophthalmischen Ausrüstung (S120) die ophthalmische Ausrüstung basierend auf dem öffentlichen Datensegment und wenigstens einem Teil des privaten Datensegments bei Empfang des privaten Schlüssels, der mit dem Teil des privaten Datensegments verknüpft ist, bestimmt wird.

14. Verfahren gemäß Anspruch 11 oder 12, wobei
im Schritt des Empfangens von Daten (S110) der Teil des Datensatzes ferner wenigstens einen Teil des privaten Datensegments bei Empfang eines privaten Schlüssels, der mit dem Teil des privaten Datensegments verknüpft ist, umfasst und
im Schritt des Bestimmens der ophthalmischen Ausrüstung (S120) die ophthalmische Ausrüstung basierend auf dem empfangenen Teil des Datensatzes, der das öffentliche Datensegment und den wenigstens einen Teil des privaten Datensegments umfasst, bestimmt wird.

## Revendications

1. Procédé, mis en œuvre par des moyens informatiques, pour fournir un ensemble de données relatives à un porteur d'un équipement ophtalmique et/ou à au moins une partie de l'équipement ophtalmique au niveau d'un serveur de données, l'ensemble de données étant destiné à être utilisé pour déterminer l'équipement ophtalmique adapté au porteur, le procédé comprenant :
- une **étape de réception d'un ensemble de données** (S10), au cours de laquelle l'ensemble de données relatives au porteur et/ou à au moins une partie de l'équipement ophtalmique est reçu d'un premier utilisateur, l'ensemble de données comprenant un segment de données privées et un segment de données publiques, le segment de données privées étant protégé par une clé privée fournissant un accès aux données du segment de données privées ;
- une **étape de stockage d'un ensemble de données** (S20), au cours de laquelle l'ensemble de données est stocké sur le serveur de données et associé à un identifiant ; et
- une **étape d'envoi d'identifiant** (S30), au cours de laquelle l'identifiant associé à l'ensemble de données stocké est envoyé à un second utilisateur,
**caractérisé en ce que** les données du segment de données privé sont cryptées avec une clé de cryptage privée, et dans lequel la clé de cryptage privée et la clé privée sont différentes.

2. Procédé, selon la revendication 1, dans lequel le procédé comprend en outre une **étape d'envoi d'un ensemble de données** (S41), au cours de laquelle le segment de données publiques et le segment de données privées sont envoyés au second utilisateur lors de la réception d'une requête comprenant au moins l'identifiant.

3. Procédé, selon la revendication 1, dans lequel le procédé comprend en outre une **étape d'envoi de segment de données publiques et de segment de données privées** (S42), au cours de laquelle le segment de données publiques est envoyé au second utilisateur lors de la réception d'une requête comprenant au moins l'identifiant, et au cours de laquelle le segment de données privées est envoyé au second utilisateur lors de la réception d'une requête comprenant au moins l'identifiant et la clé privée.

4. Procédé selon l'une quelconque des revendications précédentes, dans lequel le procédé comprend en outre une **étape de mise à jour** (S50), au cours de laquelle au moins un segment de données parmi le segment de données privé et le segment de données public est mis à jour.

5. Procédé selon l'une quelconque des revendications précédentes, dans lequel avant l'étape de réception d'un ensemble de données (S10), le procédé comprend une **étape de mesure de paramètres** (S1), au cours de laquelle on mesure au moins un paramètre relatif au porteur et/ou un paramètre relatif à au moins une partie de l'équipement ophtalmique, l'ensemble de données comprenant l'au moins un paramètre mesuré.

6. Procédé selon la revendication 5, dans lequel on mesure au moins une partie des données de l'ensemble de données à partir d'une modélisation 3D du visage du porteur et/ou à partir d'une image bidimensionnelle du visage du porteur.

7. Procédé selon l'une quelconque des revendications précédentes, dans lequel le segment de données privées comprend au moins des données relatives au nom du porteur et/ou à la date de naissance du porteur et/ou au sexe du porteur et/ou à la latéralité du porteur et/ou aux caractéristiques physiologiques du porteur et/ou à une couleur d'un iris d'au moins un des yeux du porteur et/ou à une position d'un centre de rotation d'au moins un des yeux du porteur par rapport à l'anatomie du porteur et/ou à un écart inter-pupillaire en vision de près pour les yeux du porteur et/ou à une distance de lecture pour au moins un œil du porteur et/ou à un angle d'abaissement de la direction de lecture du regard pour au moins un œil du porteur.

8. Procédé selon l'une quelconque des revendications précédentes, dans lequel l'équipement ophtalmique comprend une monture, et dans lequel le segment de données privées comprend au moins des données relatives à une distance de sommet pour un œil du porteur lorsque la monture est portée par le porteur et/ou à un angle d'enveloppement de la monture lorsque la monture est portée par le porteur et/ou à un angle pantoscopique de la monture lorsque la monture est portée par le porteur.

9. Procédé selon l'une quelconque des revendications précédentes, dans lequel le segment de données publiques comprend au moins des données relatives à une distance inter-pupillaire de vision de loin pour les yeux du porteur.

10. Procédé selon l'une quelconque des revendications précédentes, dans lequel l'équipement ophtalmique comprend une monture, et dans lequel le segment de données publiques comprend au moins une hauteur et une largeur en boxing de la monture et/ou une hauteur optique d'au moins un œil du porteur et/ou à une position d'un centre de rotation d'au moins un des yeux du porteur par rapport à la monture lorsque la monture est portée par le porteur.

11. Procédé, mis en œuvre par des moyens informatiques, pour déterminer un équipement ophtalmique adapté à un porteur en se basant au moins sur l'ensemble de données selon la revendication 1, associé à l'identifiant et stocké sur le serveur de données selon la revendication 1, le procédé comprenant :
- une **étape de réception de données** (S110), au cours de laquelle au moins une partie de l'ensemble de données relatives au porteur et/ou à au moins une partie de l'équipement ophtalmique est reçue lors de la réception d'une requête comprenant au moins l'identifiant, la partie de l'ensemble de données comprenant au moins le segment de données publiques ;
- une **étape de détermination d'un équipement ophtalmique** (S120), au cours de laquelle un équipement ophtalmique est déterminé sur la base de la partie reçue de l'ensemble de données.

12. Procédé selon la revendication 11, dans lequel le segment de données privées comprend au moins un premier segment de données privées et un second segment de données privées, le premier segment de données privées comprenant le second segment de données, le premier segment de données privées étant protégé par une première clé privée fournissant un accès aux données du premier segment de données privées, le second segment de données privées étant protégé par une seconde clé privée fournissant un accès aux données du second segment de données privées, la première clé privée étant différente de la seconde clé privée, et dans lequel au cours de l'étape de détermination d'équipement ophtalmique (S120), l'équipement ophtalmique est déterminé sur la base :
- du segment de données publiques et du premier segment de données privées lors de la réception de la première clé privée ; ou
- du segment de données publiques et du second segment de données lors de la réception de la seconde clé privée.

13. Procédé selon la revendication 11 ou 12, dans lequel
au cours de l'étape de réception de données (S110), la partie de l'ensemble de données comprend en outre le segment de données privées, et
au cours de l'étape de détermination de l'équipement ophtalmique (S120), l'équipement ophtalmique est déterminé sur la base du segment de données publiques et d'au moins une partie du segment de données privées lors de la réception de la clé privée associée à la partie du segment de données privées.

14. Procédé selon la revendication 11 ou 12, dans lequel
au cours de l'étape de réception de données (S110), la partie de l'ensemble de données comprend en outre au moins une partie du segment de données privées lors de la réception d'une clé privée associée à la partie du segment de données privées, et
au cours de l'étape de détermination de l'équipement ophtalmique (S120), l'équipement ophtalmique est déterminé sur la base de la partie reçue de l'ensemble de données comprenant le segment de données publiques et l'au moins une partie du segment de données privées.
